# EUROPEAN PATENT APPLICATION

(11) **EP 1 757 304 A1**
(43) Date of publication of application: **28.02.2007**
(21) Application number: 05090247.7
(22) Date of filing: 26.08.2005
(51) Int. Cl.: A61K 39/21, A61K 38/02, A61K 39/42, A61P 31/18

(54) **Pharmaceutical composition and its use for the prophylactic or therapeutic treatment of retroviral diseases**

(71) Applicant: Bundesrepublik Deutschland vertreten durch das Bundesminsterium für Gesundheit, dieses vertr. durch das Robert-Koch-Institut, 13353 Berlin (DE)
(72) Inventor: Denner, Joachim, 10625 Berlin (DE); Langhammer, Stefan, 10961 Berlin (DE); Fiebig, Uwe, 14473 Postdam (DE); Kurth, Reinhard, 14532 Kleinmachnow (DE)
(74) Representative: Ziebig, Marlene

(57) **Abstract**

The invention relates to a pharmaceutical combined composition comprising as active ingredients an effective amount of the retroviral transmembrane envelope protein p15E, gp41 or gp36, and an effective amount of a retroviral surface envelope protein, whereby said proteins are contained as single molecules, for inducing neutralizing antibodies which are directed against retroviral infections. The invention also relates to a method for inducing an antibody response and a method for passive immunization of a mammal by using neutralizing antibodies which are generated with said pharmaceutical composition.

SEQUENZPROTOKOLL

<110> BRD, vertreten durch das Robert-Koch-Institut

<120> Pharmaceutical composition and its use for the
      prophylactic or therapeutic treatment of retroviral
      diseases

<130> P358905EP

<140>
<141>

<160> 13

<170> PatentIn Ver. 2.1

<210> 1
<211> 9
<212> PRT
<213> Feline leukemia virus

<220>
<221> SIMILAR
<222> (1)..(9)
<223> p15E E1a

<400> 1
Leu Glu Thr Ala Gln Phe Arg Gln Leu
  1               5

<210> 2
<211> 9
<212> PRT
<213> Feline leukemia virus

<220>
<221> SIMILAR
<222> (1)..(9)
<223> p15E E1a

<400> 2
Lys Ala Leu Leu Glu Thr Ala Gln Phe
  1               5

<210> 3

<211> 13
<212> PRT
<213> Feline leukemia virus

<220>
<221> SIMILAR
<222> (1)..(13)
<223> p15E E1b

<400> 3
Ile Gln Ala Leu Glu Glu Ser Ile Ser Ala Leu Glu Lys
1               5                   10


<210> 4
<211> 11
<212> PRT
<213> Feline leukemia virus

<220>
<221> SIMILAR
<222> (1)..(11)
<223> p15E E1b

<400> 4
Ala Leu Glu Glu Ser Ile Ser Ala Leu Glu Lys
1               5                   10


<210> 5
<211> 7
<212> PRT
<213> Feline leukemia virus

<220>
<221> SIMILAR
<222> (1)..(7)
<223> p15E E2a

<400> 5
Lys Gln Arg Gln Gln Leu Phe
1               5


<210> 6
<211> 14
<212> PRT
<213> Feline leukemia virus

<220>
<221> SIMILAR
<222> (1)..(14)
<223> p15E E2a

<400> 6
Met Ala Lys Leu Arg Glu Arg Leu Lys Gln Arg Gln Gln Leu
1               5                   10

<210> 7
<211> 15
<212> PRT
<213> Feline leukemia virus

<220>
<221> SIMILAR
<222> (1)..(15)
<223> p15E E2a

<400> 7
Met Ala Lys Leu Arg Glu Arg Leu Lys Gln Arg Gln Gln Leu Phe
1               5                   10                  15

<210> 8
<211> 13
<212> PRT
<213> Feline leukemia virus

<220>
<221> SIMILAR
<222> (1)..(13)
<223> p15 E2b

<400> 8
Phe Asp Ser Gln Gln Gly Trp Phe Glu Gly Trp Phe Asn
1               5                   10

<210> 9
<211> 7
<212> PRT
<213> Feline leukemia virus

<220>
<221> SIMILAR

<222> (1)..(7)
<223> p15E E2b

<400> 9
Trp Phe Glu Gly Trp Phe Asn
1               5

<210> 10
<211> 17
<212> PRT
<213> Feline leukemia virus

<220>
<221> SIMILAR
<222> (1)..(17)
<223> p15E isu domain

<400> 10
Leu Gln Asn Arg Arg Gly Leu Asp Ile Leu Phe Leu Gln Glu Gly Gly
1               5                   10                  15

Leu

<210> 11
<211> 20
<212> DNA
<213> Künstliche Sequenz

<220>
<223> Beschreibung der künstlichen Sequenz: internal
      probe in real time PCR

<400> 11
ttaagcacct gggccccggc                                                20

<210> 12
<211> 25
<212> DNA
<213> Künstliche Sequenz

<220>
<223> Beschreibung der künstlichen Sequenz:
      FeLV-specific primer

<400> 12
tcaagtatgt tcccatgaga tacaa                                          25

<210> 13
<211> 22
<212> DNA
<213> Künstliche Sequenz

<220>
<223> Beschreibung der künstlichen Sequenz:
      FeLV-specific primer

<400> 13
gaaggtcgaa ctctggtcaa ct                                             22

## Description

The invention relates to a pharmaceutical combined composition comprising as active ingredients an effective amount of the retroviral transmembrane envelope protein p15E, gp41 or gp36, and an effective amount of a retroviral surface envelope protein, whereby said proteins are contained as single molecules, for inducing neutralizing antibodies which are directed against retroviral infections. The invention also relates to a method for inducing an antibody response and a method for passive immunization of a mammal by using neutralizing antibodies which are generated with said pharmaceutical composition.

Immunization is the most effective method to prevent diseases caused by infectious agents. Although the development of vaccines against HIV, the retrovirus that causes AIDS, is one of the major task of present vaccinology, all attempts have failed until now. Approximately 40 million individuals world wide were living with HIV in 2003, and 28 million have died since the pandemic began. Present therapies are mainly based on combinations of inhibitors of the viral reverse transcriptase and of the protease. Fusion inhibitors, such as T20 that corresponds to a conserved domain of the transmembrane envelope protein gp41 of HIV-1 and is able to prevent infection, have been included in combination therapies. Although there are therapies that prevent or delay the onset of AIDS, there is currently no cure available for the infection. In addition, passive immunization using broadly neutralizing monoclonal antibodies such as 2F5 and 4E10 derived from HIV infected patients have been applied to treat HIV infection. However, numerous attempts to induce the corresponding neutralizing antibodies using recombinant gp41 or synthetic peptides have failed.

It is also known that three types of feline leukemia virus (FeLV) vaccines are currently available: inactivated whole virus preparations, inactivated mixed subunit preparations from FeLV-infected tissue culture filtrate and recombinant FeLV proteins. In detail, three vaccines are composed of inactivated whole virus, two are gp70 subunit vaccines and two are recombinant vaccines. The commercially available vaccines containing inactivated FeLV subunit preparations are Fevaxyn, Leucocine and Leukocell2. Leucogen (Virbac) is an example of a recombinant vaccine comprising recombinant non-glycosylated surface envelope protein p45. However, FeLV vaccines presently in wide use are generally poor inducers of virus neutralizing antibodies. None of the vaccines regularly induce virus neutralizing antibodies following vaccination: such antibodies are usually detected after recovery from challenge only. Therefore, none of the seven commercial FeLV vaccines currently available in the USA and Europe provide 100% protection against infection, and there is only limited scientific data concerning long-term duration of immunity after vaccination.

It was recently shown that neutralizing antibodies can be induced which are specific for the transmembrane envelope protein p15E of porcine endogenous retrovirus (Fiebig et al. (2003) Virology 307, 406-413) and of FeLV-A in goats and rats (Langhammer et al. (2005), Vaccine 23, 3341-3348). The latter can exhibit a neutralization capacity of up to 99 % at a serum dilution of 1:5.

EP 0 247 904 A1 discloses the induction of antibodies by a fusion protein containing the complete p15E transmembrane envelope protein and the gp70 surface envelope protein. gp70 is not glycosylated when the viral gp70 genes are expressed in E. coli. The physical form of the recombinant antigen used to immunize cats can be either aggregated or non-aggregated. The composition could be effective in producing an active immune reaction to protect the animals against an exposure to FeLV or immunologically related viruses. It is unclear whether neutralizing antibodies have been induced or not. Furthermore, the aggregated form makes the handling difficult.

Therefore, the technical problem forming the basis of the present invention is to provide a pharmaceutical composition which has an improved efficacy in neutralizing retroviruses and is able to protect mammals safely against retroviral infections.

The present invention solves this problem by providing a pharmaceutical combined composition comprising as active ingredients an effective amount of the retroviral transmembrane envelope protein p15E, gp41 or gp36, and an effective amount of a retroviral surface envelope protein, or parts thereof, or the DNA encoding said proteins, whereby said proteins are contained as single molecules.

Surprisingly, it has been found that higher titers of neutralizing antibodies are induced by the combined immunization with p15E and a surface envelope protein, such as for instance the non-glycosylated p45, in comparison to responses using either p15E or the surface envelope protein alone. Neutralization relates to a mechanism which prevents or inhibits the infection and propagation of a retrovirus, i.e. the immune system is activated by the inventive pharmaceutical composition. The neutralizing antibodies are especially directed against the viral structures which induced them. Both active ingredients are recognized by distinct antibody fraction. The virus infection is prevented by the direct interaction of the neutralizing antibodies with both the retroviral transmembrane envelope protein and the retroviral surface envelope protein. Although the total titer of binding antibodies as well as the single titers of binding antibodies to the respective active substances remain approximately constant, the neutralization capacity is unexpectedly enhanced. The inventive composition generates a retroviral protection of every subject of such species capable of being immunized. In addition, the complete anti-viral protection achieved herewith is of clear advantage over the partial immunization of a mammal subset if using the components alone. Particularly, the inventive pharmaceutical composition causes high titers of neutralizing antibodies within short periods of immunization along with an effective reduction of the number of retroviruses. The high neutralizing antibody titers are reflected by a high dilution of serum which is obtained after immunization and used in neutralization assays. Simultaneously, adverse effects which could be caused by other serum components are largely reduced due to their diluted presence. The inventive composition is also characterized by advantageous physical/ chemical features of the single molecules, such as high expression rate, solubility, stability and/or size enabling a convenient handling.

The terms "effective amount" or "effective dose" or "dose" are interchangeably used herein and denote an amount of a pharmaceutical compound having a prophylactically or therapeutically relevant effect on a retroviral caused disease or pathological conditions. A prophylactic effect prevents the infection with a retrovirus after the infiltration of single viral representatives such that the subsequent propagation of the virus is strictly diminished, or it is even completely inactivated. A therapeutically relevant effect relieves to some extent one or more symptoms of a retroviral disease or returns to normal either partially or completely one or more physiological or biochemical parameters associated with or causative of the disease or pathological conditions. The determination of an effective amount is a routine exercise in the pharmaceutical arts taking various physical parameters, such as weight, age and the like into account, and is best determined by the attending clinician.

The retroviral transmembrane envelope protein and the surface envelope protein can be combined to the inventive pharmaceutical composition without viral species restriction. It is possible that a specific p15E or a specific surface envelope protein can generally act as enhancer in the meaning of the invention. The principle underlying the present synergistic effect is not known. Preferably, the proteins of the pharmaceutical composition are of the same retroviral origin. It guarantees an antibody induction which is specifically and exclusively directed against the same retrovirus target, thereby minimizing the probability of cross-reactivity.

The family of transmembrane envelope proteins have a homologous primary structure. The transmembrane envelope proteins, such as p15E, gp41 or gp36 are associated with the membrane by at least one membrane passage, further comprising at least one fusion domain and at least two alpha-helical structures, NHR and CHR (N-terminal helical region and C-terminal helical region, respectively). The active ingredient p15E is obtained from any retrovirus comprising a membrane which is associated with p15E, preferably a gammaretrovirus. It is more preferably obtained from FeLV, PERV or KoRV, most preferably FeLV. The active ingredients gp41 or gp36 are obtained from any retrovirus comprising a membrane which is associated with gp41 or gp36, preferably a lentivirus, more preferably HIV-1 or HIV-2.

In an embodiment of the invention, the retroviral transmembrane envelope protein comprises a part thereof. It is preferred that the part comprises the ectodomain of p15E, gp41, gp36 or any other retroviral transmembrane envelope protein, preferably of p15E, gp41 or gp36, whereby the ectodomain consists of two alpha-helical structures and a cysteine-cysteine-loop. More preferably, the part is the ectodomain or variants, mutants, parts of the ectodomain or homologous sequences having the same function. A couple of methods are known to the skilled artisan to generate equivalent ectodomains, i.e. proteins which are analog in function to those of the inventive teaching. Therefore, the invention also contains the aforementioned modifications. For example, mutants can be generated by substitution, deletion, insertion, translocation, inversion and/or addition of at least a single amino acid. It is known that certain amino acids exhibit similar physicochemical characteristics making the substitution among each other possible. Variants of the ectodomain can be arise from modifications, such as alkylation, arylation or acetylation of at least a single amino acid, from incorporation of enantiomers and/or from fusion of the ectodomain with a single or multiple amino acids, a peptide or a protein. It is preferred in the meaning of the invention that the ectodomain is fused to a purification tag for affinity chromatography. Parts of the ectodomain relates to a restriction to those regions which are sufficient for the expression of a specific function. All alterations are inevitably limited by the requirement of preserving the function. However, the parts of the ectodomain can be very small due to the characterization of epitope which also induce neutralizing antibodies as peptides. In the meaning of the invention, it is to be clearly distinguished between ectodomain parts of any size and homologous sequences which homology is related to the entire ectodomain. Preferably, the homology between a natural ectodomain and a derivative thereof having the same features amounts to at least 60%, more preferably 75%, most preferably 90%. Similarly, the homology is to be considered if the aforementioned part of any size is altered to a variant or mutant. In addition, several techniques are described in prior art to generate non-homologous peptides with the same function. All peptide derivatives which are developed on the basis of the present ingredients by such procedures are covered by the present teaching if solving the problem of the invention.

In general, transmembrane envelope proteins comprise an ectodomain, an anchor domain (also termed membrane passage), a fusion domain and a cytoplasmatic portion, whereby the ectodomain comprises two alpha-helical structures and a cysteine-cysteine-loop. It has been found that membrane passage, fusion domain and cytoplasmatic portion are dispensable for inducing neutralizing antibodies. The ectodomain contains highly conserved regions which do not mutate during virus replication, thereby representing the principal antibody target. These regions termed epitopes E1 and E2 have been identified for several ectodomains. The ectodomain is characterized by the induction of neutralizing antibodies in several species and a better solubility compared to the total transmembrane envelope protein. Due to its small size, the ectodomain is cost-efficiently produced. In the scope of the invention it is even sufficient that p15E, gp41 or gp36 comprise the essential epitope regions to generate the desired immune reaction of 2F5/4E10-like antibodies.

In a preferred embodiment, the ectodomain comprises the amino acid sequences E1a (LETAQFRQL), E1b (IQALEESISALEK), E2a (KQRQQLF) and/or E2b (WFEGWFN) which are constituents of the FeLV p15 ectodomain. Epitope mapping of antibodies induced by simultaneous immunization with a surface envelope protein, such as p45 (Leucogen), and p15E reveal the same pattern of response as described after immunization with p15E alone. The same epitopes are detected, two of them are located at the N-terminus of the ectodomain (designated epitope E1 a and E1 b) and two others at the C-terminus of the ectodomain (designated epitope E2a and E2b). The presence of both E1 and E2 seems to be crucial. Furthermore, the general proximity and immunogenicity suggests that the E2a and E2b epitopes represent the functional equivalents of the 2F5 and 4E10 epitopes, respectively. Sera from rats immunized with FeLV p15E recognize a 4E10 equivalent and/or a 2F5 equivalent in the C-terminal end and E1a and/or E1b in the N-terminal end. Simultaneous immunization of both antigens increased also the recognition of an epitope in p15E crucial for neutralization (E1 b).

In another embodiment of the present invention, p15E comprises an epitope E2 between membrane passage and C-terminal helical region, whereby the E2 epitope is selected from a region between membrane passage and C-terminal helical region of a retroviral transmembrane envelope protein which is not identical to p15E. It is known from DE 103 39 966 A1 that neutralizing antibodies are exclusively induced against such an epitope if presented in the context of another transmembrane envelope protein. The patent is incorporated by reference in its entirety including all figures and drawings herein. The retrovirus to be attacked is determined by the epitope E2 which is located between membrane passage and C-terminal helical region (CHR). In the meaning of the invention, the location or selection "between" something includes adjacent regions, i.e. membrane passage and CHR, which can be at least partly a component of the epitope E2. The original epitope E2 of p15E can be replaced by the epitope E2 of another retroviral transmembrane envelope protein, whereby the initial primary structure is partially or completely removed. Suitable techniques are known to the skilled artisan, such as substitution, insertion and addition. The epitopes E2 show a homology concerning their secondary and/or tertiary structure enabling sequence allocation and the specific incorporation at a certain position. The occurrence of cross-reactivity is considered as unlikely. For example, an engineered transmembrane envelope protein, which backbone is derived form p15E and which epitope E2 is derived from HIV-1 gp41, exclusively affects HIV-1. Cross-reactivity might be possible due to the conservation of the epitope E2 in homologous transmembrane envelope proteins of closely related retroviruses, e.g. PERV and FeLV.

The epitope E2 to be incorporated can be derived from a transmembrane envelope protein of any retrovirus mentioned in the course of the present specification. Preferred retroviruses comprising a transmembrane envelope protein for E2 selection in the meaning of the invention are lentiviruses, more preferably HIV-1 or HIV-2. Preferred transmembrane envelope proteins for the epitope E2 selection in the meaning of the invention are gp41, p15E, gp20, gp21, gp30, gp36, gp37, gp40, gp45 and gp160. Secondary and tertiary structures are known for all mentioned and claimed transmembrane envelope proteins making the E2 selection possible for those skilled in the art.

In a preferred embodiment of the present invention, p15E comprises the epitope E2 of HIV-1 gp41.

The surface envelope protein can be originated from a retrovirus selected from the group of FeLV, PERV, KoRV, HIV-1, HIV-2, BIV, CAEV, EIAV1, FIV, OMVV, SIVmac, SIVcpz, VILV, RSV, ALV, JSRV, SRV, GALV, MuLV, BLV, HTLV-1 and HTLV-2. The retroviruses are abbreviated as follows:
- BIV: Bovine immunodeficiency virus
- CAEV: Caprine arthritis encephalitis virus
- EIAV1: Equine infectious anemia virus
- FIV: Feline immunodeficiency virus
- OMVV: Ovine maedi visna virus
- SIVmac: Simian immunodeficiency virus form macac
- SIVcpz: Simian immunodeficiency virus from chimpanzee
- VILV: Visna lenti virus
- HIV-1: Humane immunodeficiency virus type 1
- HIV-2: Humane immunodeficiency virus type 2
- RSV: Rous sarcoma virus
- ALV: Avian leucosis virus
- JSRV: Jaagsiekte sheep retrovirus
- SMRV: Squirrel monkey retrovirus
- SRV: Simian retrovirus
- GALV: Gibbon ape leukemia virus
- MuLV: Murine leukemia virus
- FeLV: Feline leukemia virus
- KoRV: Koala retrovirus
- PERV: Porcine endogenous retrovirus
- BLV: Bovine leukemia virus
- HTLV-1: Humane T-cell lymphotropic virus type 1
- HTLV-2: Humane T-cell lymphotropic virus type 2

Preferably, the surface envelope protein is originated form a gammaretrovirus or a lentivirus, more preferably FeLV, PERV, KoRV, HIV-1 or HIV-2, most preferably FeLV, PERV or KoRV. It is particularly preferred to select both the surface envelope protein and p15E from the same retroviral origin which is FeLV, PERV or KoRV. If engineered p15E is part of the inventive composition, the antibody response is determined by the epitope E2, thereby preferring the combination with the surface envelope protein of that retrovirus the epitope E2 of a transmembrane envelope protein is derived from. In this case, p15E acts as backbone and conformational scaffold, whereas suited combinations of E2 and surface protein can be chosen among the diversity of retroviruses.

Of course, it is possible that variants, mutants, parts of the surface envelope protein or homologous sequences having the same features represent an active ingredient of the present invention. The prior teaching of the present specification concerning the ectodomain and derivatives thereof is considered as valid and applicable without restrictions to alterations of the surface envelope protein if expedient.

In an embodiment of the invention, the surface envelope protein is gp70, gp120 or an unglycosylated form thereof. Either the glycosylated or the non-glycosylated form increase the neutralizing titer significantly if combined with p15E. The unglycosylated form is preferred, and it can be advantageously produced in prokaryotic expression systems at low costs. In a preferred embodiment of the invention, the surface envelope protein is p45 which corresponds to the unglycosylated form of gp70. Its sequence has always to correlate with the virus against the immunization is directed, preferably gp70 or p45 from FeLV, PERV or KoRV.

The active ingredients of the pharmaceutical combined composition are obtained from natural sources. It is possible to gather total proteins form the retrovirus, or parts thereof from the retroviral protein, respectively. Preferably, the ingredients are recombinantly expressed and purified. Therefor, one or both ingredients can be fused with a tag for affinity chromatography, such as Strep-tag, His-tag, GST-tag, Arg-tag or the calmodulin binding protein, preferably the calmodulin binding protein (CBP). CBP binds to a calmodulin resin, e.g. to be used as column matrix. The column is loaded with the protein suspension and all components lacking CBP are immediately eluted. After removal of unspecific binders by washing steps, the CBP fused ingredient are removed from the column. The tag does not affect the induction of neutralizing antibodies of the ingredients. Alternatively, the DNA encoding the protein sequences can be obtained, amplified, altered or synthesized with techniques known to the skilled artisan. Subsequently, the DNA can be introduced into a vector and transcribed and translated in cells.

Object of the present invention is also the use of an effective amount of the retroviral transmembrane envelope protein and an effective amount of a retroviral surface envelope protein, or parts thereof, or the DNA encoding said proteins, whereby said proteins are contained as single molecules, for the manufacture of a pharmaceutical combined composition for the prophylactic or therapeutic treatment and/or monitoring of retroviral diseases, preferably retroviral immune diseases. The ingredients can be either administered to prevent the infection of a mammal with a retrovirus and the outbreak of the disease in advance, or to treat the disease caused by the infectious agent. Particularly, later stages of virus internalization can be prevented. Herein, monitoring is considered as kind of treatment provided that the pharmaceutical combined composition is sequentially administered, e.g. in order to booster the immune response and eradicate the retrovirus and the arisen symptoms completely. Numerous retroviral diseases can be successfully combated by applying the inventive composition, such as leukemia and immunodeficiency caused by FeLV, lymphoma and immunodeficiency caused by KoRV, or AIDS caused by HIV. Another example is PERV representing a clear threat during xenotransplantation of pig cells or organs because these viruses are present in all pigs and can infect human cells. The prior teaching of the present invention and embodiments thereof is considered as valid and applicable without restrictions to the use of the pharmaceutical combined composition if expedient.

In an embodiment of the invention, the aforementioned active substances are used for the manufacture of a pharmaceutical combined composition for the prophylactic or therapeutic treatment and/or monitoring of a FeLV infection. FeLV is a gammaretrovirus, closely related to PERV, which induces fatal leukaemia, lymphoma and immunosuppression associated with opportunistic infections in infected cats. The cat/FeLV system additionally helps to address the question of whether PERV p15E can induce protective neutralizing antibodies since there is no animal that can be artificially infected with PERV.

The composition of the invention is produced in a known way using the usual solid or liquid carriers, diluents and/or additives and the common adjuvants for pharmaceutical engineering and with an appropriate dosage depending on the intended mode of application. These pharmaceutically acceptable excipients comprise salts, buffers, fillers, chelating agents, antioxidants, solvents, bonding agents, lubricants, tablet coatings, flavor additives, flavors, preservatives and suspending agents. In the meaning of the invention, an adjuvant denotes every substance which enables, intensifies or modifies a specific immune response against the inventive composition if administered simultaneously, contemporarily or sequentially. Known adjuvants for vaccines are for example aluminum compositions, such as aluminum hydroxide or aluminum phosphate, saponins, such as QS21, muramyldipeptide or muramyltripeptide, proteins, such as gamma-interferon or TNF, M59, squalen or polyols. The co-application of egg albumin in complete Freund's adjuvant also increases cell-mediated immunity, thereby supporting the effect of neutralizing antibodies. Preferred adjuvants are Freund's adjuvant, Leucogen adjuvant containing Quil-A and aluminum hydroxide, or Montanide, such as Montanide ISA 720. The amount of excipient material that is combined with the active ingredient(s) to produce a single dosage form varies depending upon the host treated and the particular mode of administration.

Possible formulations of the inventive composition are those forms which are suitable for oral administration, such as tablets, film tablets, lozenges, capsules, pills, powders, solutions, dispersions, suspensions or depot forms thereof, for transdermal administration, such as solutions, suspensions, creams, emulsions or band-aids, for parental administration, such as suppositories, and for intravenous infusion, subcutaneous injection or intramuscular administration, examples for the latter three are solutions and suspensions.

In a preferred embodiment of the invention, said composition is a vaccine or vaccine-adjuvant. In accordance with the present invention, the term vaccine composition relates to any composition which can be used as a vaccine. A vaccine means a therapeutic or prophylactic use of the medicament which attacks retroviruses. The vaccination is advantageously performed in such a way that an active protection is provided in the mammal. The initial immunization can be boostered by subsequent inoculations. Furthermore, the inoculation can be administered before or following an infection once or several times acting as therapy. The forms or methods for manufacturing vaccine compositions according to the present invention are not particularly limited, and a composition in a desired form can be prepared by applying a single method available in the field of the art or methods in an appropriate combination. For the manufacture of a vaccine composition, aqueous media such as distilled water for injection and physiological saline, as well as one or more kinds of pharmaceutical additives available in the field of the art can be used. For example, buffering agents, pH adjusting agents, solubilizing aids, stabilizing agents, soothing agents, antiseptics, and the like can be used, and specific ingredients thereof are well known to those skilled in the art. The vaccine composition can also be prepared as a solid preparation such as a lyophilized preparation, and then prepared as an injection by adding a solubilizing agent such as distilled water for injection before use. Depending upon the manner of introduction, the compounds may be formulated in a variety of ways as discussed below. The concentration of therapeutically active compound in the formulation may vary from about 0.1 to 100 wt %. The vaccine composition may be administered alone or in combination with other treatments. In a preferred embodiment, the vaccine compositions are in a water-soluble form, such as pharmaceutically acceptable salts, which is meant to include both acid and base addition salts. The vaccine compositions can be prepared in various forms, such as injection solutions, suspensions and the like. Preferably, it is used as an injection solution. The vaccine compositions may also include one or more of the following: carrier proteins, such as serum albumin, buffers, stabilizing agents, coloring agents and the like. Additives are well known in the art, and are used in a variety of formulations.

The pharmaceutical composition can also contain, if desired, other retrovirus-attacking agents. The inventive composition can be used as vaccine adjuvant to enhance the protection afforded by animal or human vaccines that are considered weak, i.e. by providing diminished protection in terms of level, extent, and/or duration. The ingredients as vaccine-adjuvant will normally be administered separately from the vaccine, although it may be administered in combination with the vaccine as well. Administration of the inventive composition as vaccine-adjuvant can be subcutaneous, intravenous, parenteral, intramuscular, or in any other acceptable method. Preferably, the vaccine-adjuvant is administered prior to the administration of the vaccine and at the same site where the vaccine is to be administered. The formulations and pharmaceutical compositions contemplated by the above dosage forms can be prepared with conventional pharmaceutically acceptable excipients, and by using conventional techniques. Other adjuvants may be administered either with the vaccine or together with the inventive pharmaceutical combined composition.

It will be understood that the specific dose level, frequency and period of administration of any particular mammal will depend upon a variety of factors including the activity of the specific compound employed, the age, body weight, general health, sex, diet time of administration, route of administration, rate of excretion, drug combination and the severity of the specific therapy.

The active ingredients of the inventive combined composition can be simultaneously administered as fixed composition, i.e. being a single pharmaceutical formulation which contains both ingredients. It is prepared for example as injection or infusion solution, or lyophilized form thereof, which is filled in ampoules. The fixed composition of the lyophilized form guarantees a simple and secure handling. It is solved in the ampoule by adding an ordinary pharmaceutical injection agent and administered intravenously. The reconstitution solution can be part of the combination package.

It is also possible to provide the transmembrane envelope protein and the surface envelope protein as single pharmaceutical compositions which are either mixed prior administration or sequentially administered. Usually, a single packing unit comprising two boxes is offered, whereby the first one represents a suitable pharmaceutical form for the transmembrane envelope protein (injection or infusion solution, lyophilized form) and the second one represents a suitable pharmaceutical form for the surface envelope protein (injection or infusion solution, lyophilized form). This free combination is of benefit by individually allotting an effective amount of the transmembrane envelope protein and an effective amount of a surface envelope protein to the mammal. Another possibility is the provision of single preparations of the transmembrane envelope protein and the surface envelope protein being independent medicaments. The single preparations are converted to contain the required amounts of ingredient for the inventive combination. Corresponding instructions are given at the package insert concerning the combined administration of the respective medicament. Preferably, the inventive composition is sequentially administered and at different sites of the mammal. Contrary, a decrease of binding antibody titer is recognized while simultaneously administered, whereas the neutralization capacity is not affected.

Another object of the present invention are neutralizing antibodies produced by immunization with the inventive pharmaceutical combined composition. For the production of neutralizing antibodies at least one inventive composition is exposed to a mammal that it is capable to induce antibodies which are directed against the active substances of the composition and obtained by routine procedures known to those skilled in the art. Any mammal is chosen which produces high antibody titers. The neutralizing antibodies are used for prophylactic or therapeutic treatment and/or monitoring of retroviral diseases, preferably a FeLV infection. The neutralizing effect of the antibodies is demonstrated by inhibiting either the viral infection, the formation of syncytiums or the fusion between virus and target membrane, or everything thereof, or by reducing or stabilizing the propagation rate of a retrovirus in a mammal. The effect of the antibodies is not restricted to the elimination of a retrovirus, but comprises the entire spectrum of advantageous effects in prophylaxis and therapy.

The present invention also relates to a method for treating a retroviral disease, preferably a FeLV infection, wherein as active substances an effective amount of the retroviral transmembrane envelope protein p15E and an effective amount of a retroviral surface envelope protein, or parts thereof, or the DNA encoding said proteins, are administered to a mammal in need of such treatment, whereby said proteins are administered as single molecules. However, the transfer of the retrovirus to new hosts is not be excluded due to the viral variability. The prior teaching of the present invention and embodiments thereof is considered as valid and applicable without restrictions to the method of treatment if expedient.

Furthermore, the invention relates to a method for inducing an antibody response, wherein the inventive pharmaceutical combined composition is administered to a mammal, thereby inducing the production of neutralizing antibodies. It has been shown that the best antibody response concerning the neutralization capacity is obtained by the immunization of that mammal being the natural host species for the retrovirus from which the ingredients of the pharmaceutical combined composition are derived. For example, after demonstrating the induction of neutralizing antibodies in rats and goats immunized with the transmembrane envelope protein p15E of FeLV, cats have been immunized with the same antigen. High titers of neutralizing antibodies specific for FeLV are induced and epitope mapping reveals a pattern of recognition similar to that seen following immunization of rats and goats. These epitopes are highly related to epitopes recognized after immunization with PERV p15E and to epitopes recognized by neutralizing antibodies in patients infected with HIV-1. Tolerance against these epitopes is not induced. The skilled artisan can set the concentration and the mode of application by routine experiments as already mentioned in the course of the specification. The administration is performed prophylactically or therapeutically. Preferably, the composition is injected. It is the intention of a prophylactic inoculation to prevent the infection with a retrovirus after the infiltration of single viral representatives, e.g. into a wound, such that the subsequent propagation of the virus is strictly diminished, or it is even completely inactivated. If an infection of the patient is already given, a therapeutic induction of an immune response is performed in order to inactivate the retrovirus being present in the body or to stop its propagation.

A method for passive immunization of a mammal is still another object of the present invention, wherein the antibodies induced by administering the inventive pharmaceutical combined composition to a mammal are separated and administered to a further mammal. In the meaning of the invention, "further mammal" refers to a mammal which is identical to the species or foreign to the species of that mammal having induced said antibodies, whereas the same mammal is disclaimed. The presence of neutralizing antibodies in cats recovering from natural FeLV infection clearly correlates with resistance to subsequent infection, and passive transfer of antibodies can protect other animals as well. In addition, monoclonal antibodies can be produced and used, such as in humans following humanization. In doing so, it is also possible to obtain antibody-producing cells from inoculated or infected individuals which neutralizing antibodies are directed against the inventive composition and applied as monoclonal antibodies during passive immunization. That mode of immunization does not cause a patient's own immune reaction to certain viruses, but the antibodies are introduced in the body as healing sera. The approach aims at a prompt effect, i.e. to cure the given infection sickness as quickly as possible or to protect against a viral infection immediately. Vaccination schedules are known to those skilled in the art and can be easily adapted to specific retroviruses which are to be attacked by the combined composition of the present invention. Preferably, monoclonal antibodies are used for passive immunization. Their use in a combination therapy is of special benefit.

### EXAMPLES

The following examples is provided by way of illustration and not by way of limitation. Within the example, standard reagents and buffers that are free from contaminating activities (whenever practical) are used.

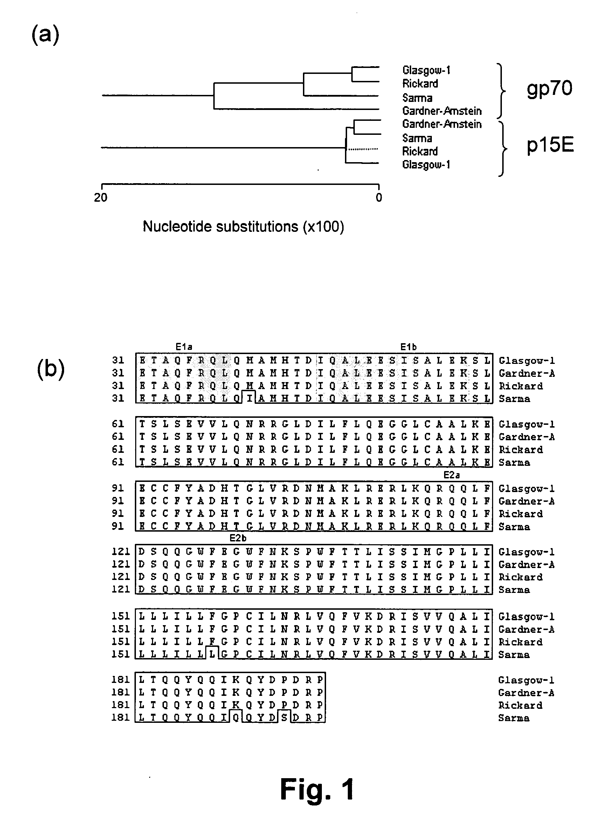

Fig. 1: Comparison of p15E of different FeLV. (a) Unrooted phylogenetic tree of gp70 and of p15E of the FeLV strains Glasgow-1, Rickard, Sarma (FeLV-A) and Gardner-Arnstein (FeLV-B). (b) Alignment of epitopes designated E1a, E2a, E1b and E2a identified after immunization of rats with the recombinant ectodomain of p15E of FeLV (indicated in gray) in different FeLV-A strains.

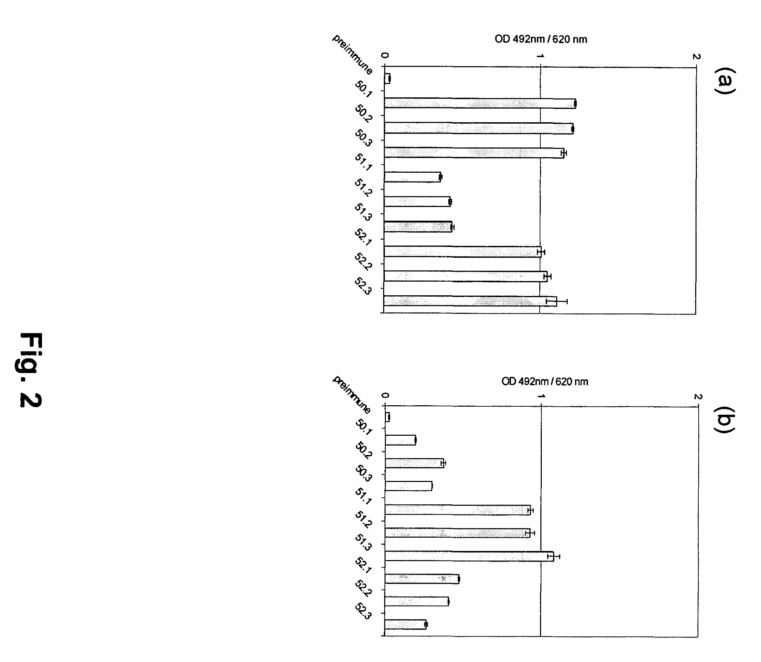

Fig. 2: ELISA reactivity of rat antisera induced by immunization with 500µg p15E alone (group 50), with 100µg Leucogen alone (group 51) and with a combination of both antigens (group 52). As antigen in the ELISA FeLV-A p15E (a) or Leucogen (b) were used.

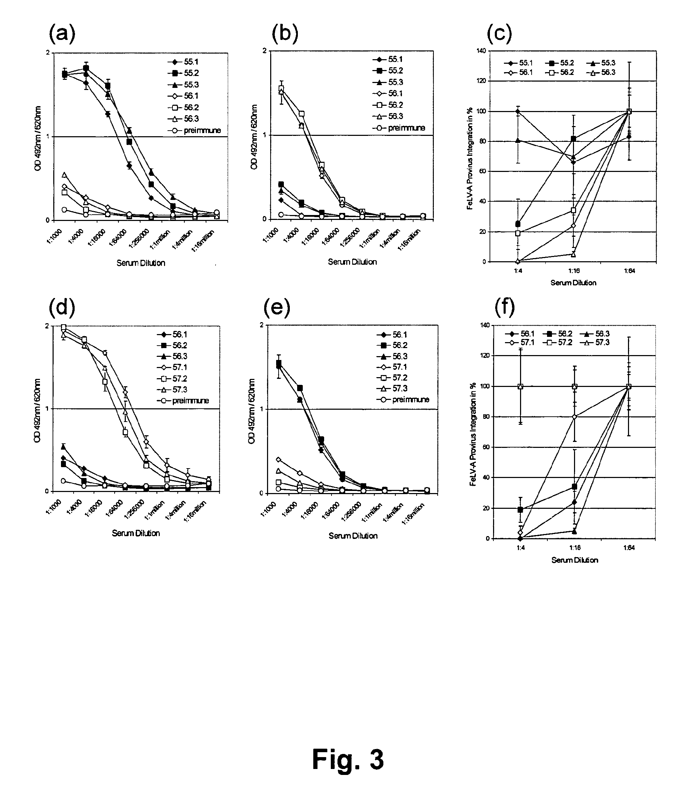

Fig. 3: ELISA reactivity (a, b, d, e) and neutralizing activity (c, f) of rat antisera induced by immunization with 500µg p15E alone (group 55), with 100µg p15E alone (group 57) and with 100µg Leucogen alone (group 56). As antigen in the ELISA FeLV-A p15E (a, d) or Leucogen (b, e) were used.

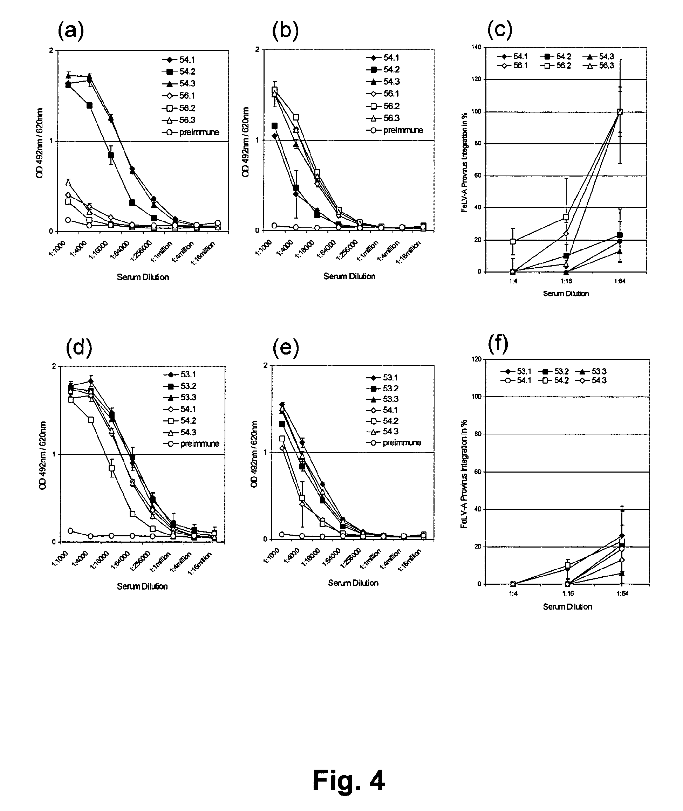

Fig. 4: ELISA reactivity (a, b, d, e) and neutralizing activity (c, f) of rat antisera induced by immunization with 500µg p15E in combination with 100µg Leucogen in a single injection site (group 54) or in two different injection sites (group 53) and with Leucogen alone (group 56). As antigen in the ELISA FeLV-A p15E (a, d) or Leucogen (b, e) were used.

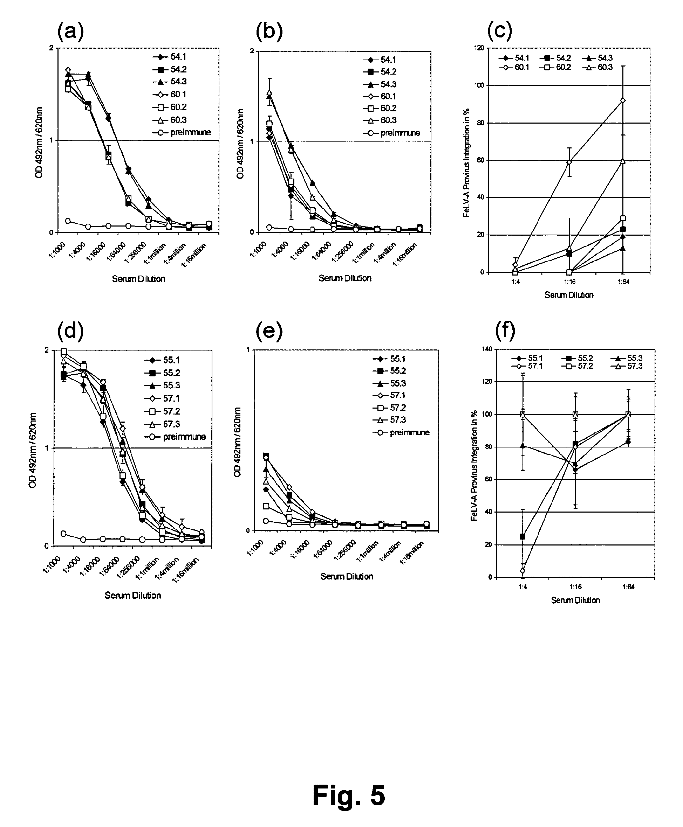

Fig. 5: ELISA reactivity (a, b, d, e) and neutralizing activity (c, f) of rat antisera induced by immunization with 500µg p15E in combination with 100µg Leucogen in a single injection site (group 54), with 100µg p15E in combination with 100µg Leucogen in a single injection site (group 60), with 500µg p15E alone (group 55) and with 100µg p15E alone (group 57). As antigen in the ELISA FeLV-A p15E (a, d) or Leucogen (b, e) were used.

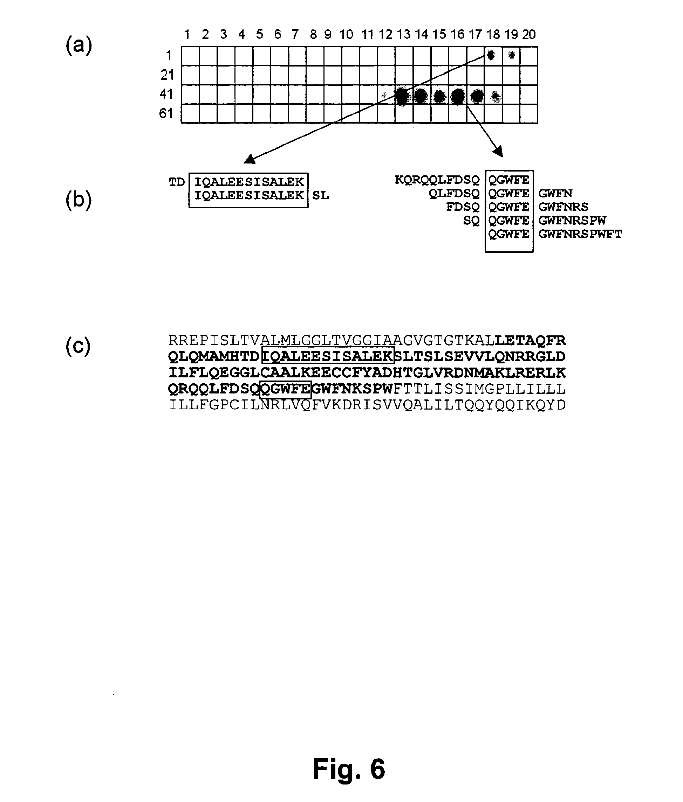

Fig. 6: Epitope mapping of the neutralizing rat antiserum 54.2 obtained after immunization with recombinant p15E of FeLV-A. (a) Result of the dot blot ECL method using overlapping peptides and in (b) sequence alignment of the recognized peptides. (c) Entire sequence of p15E of FeLV-A is given, the sequence of the recombinant protein used for immunization is printed in bold, and the epitopes are framed.

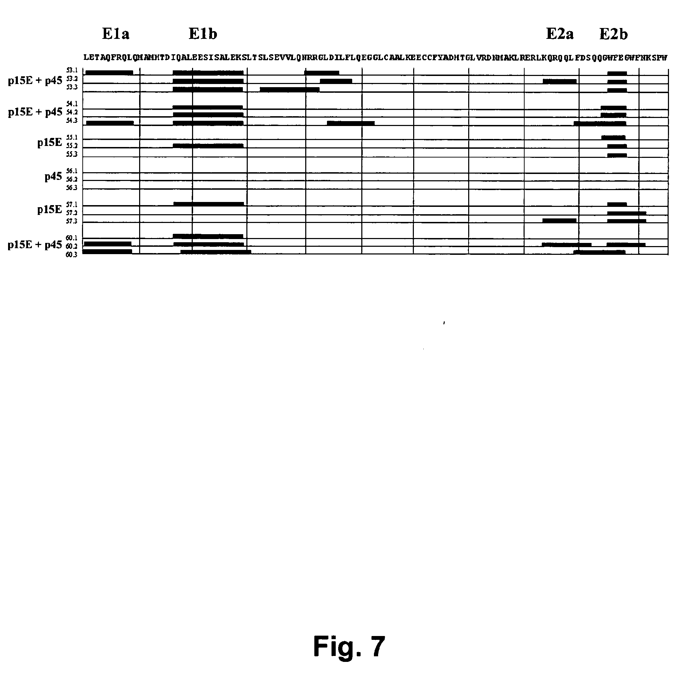

Fig. 7: Summary of the specific epitope mapping of all antisera obtained after immunization with p15E, Leucogen or both, p15E and Leucogen. The sequence of the FeLV-A p15E ectodomain and the animal number and the antigen(s) used for immunization are given, epitopes are framed.

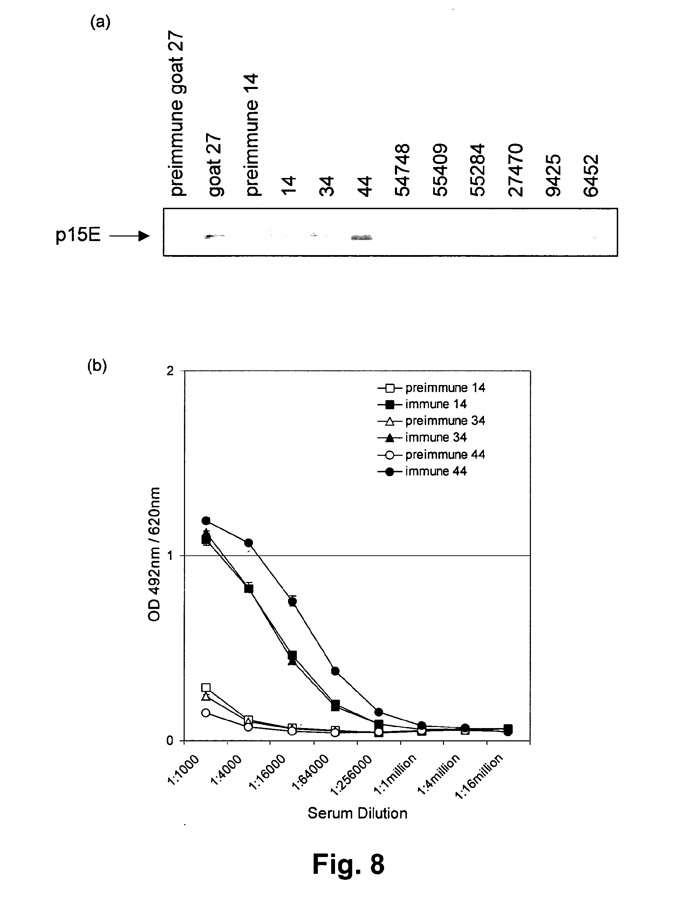

Fig. 8: (a) Western blot analysis of cat antisera following immunization with FeLV-A p15E (14, 34 and 44) and of sera from FeLV-infected cats (54748-6452). The same recombinant p15E used for immunization was used as antigen. Lane 1 shows the preimmune serum of goat 27, lane 2 the corresponding immune serum. Only one preimmune cat serum is shown (#14, lane 3) (b) ELISA reactivity of cat sera induced by immunization with 500µg p15E in comparison with the corresponding preimmune sera. Recombinant p15E was used as antigen.

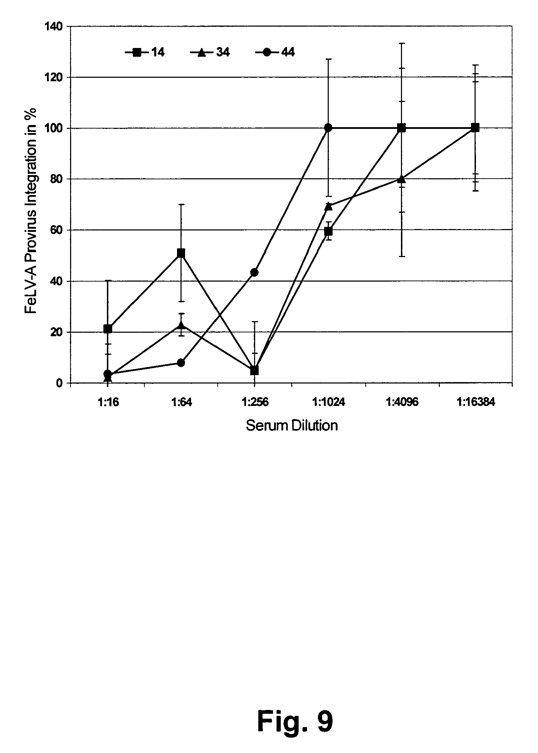

Fig. 9: Neutralizing activity of cat antisera after the second immunization with 500µg p15E. Infection was measured as provirus integration by real-time PCR. Percent of provirus integration was obtained by comparing antisera with the corresponding preimmune sera.

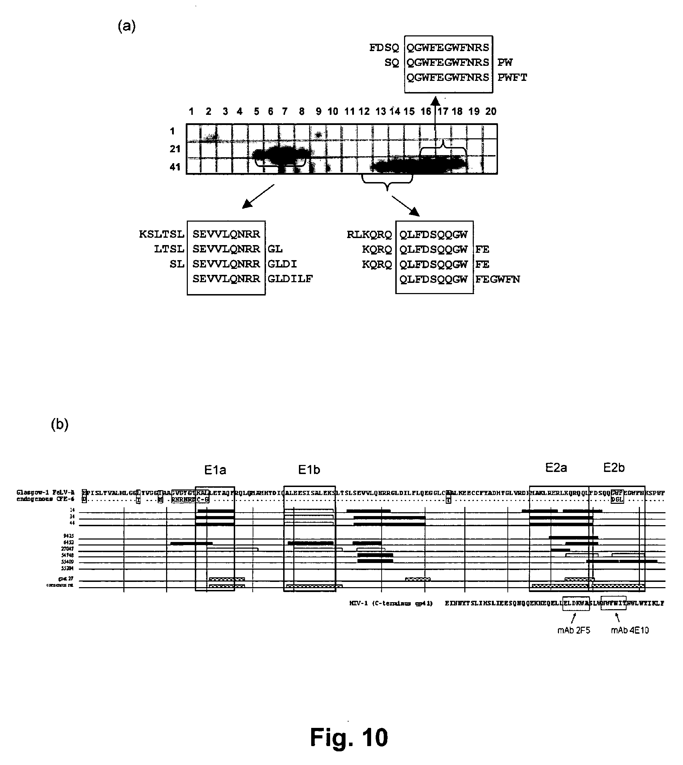

Fig. 10: Epitope mapping using sera from immunized and FeLV-infected cats. (a) As an example, binding of serum from FeLV-infected cat #55409 to a pepspot membrane carrying overlapping peptides is shown and epitopes are identified. (b) Summary of the epitopes identified recognized by each serum. Sequences corresponding to the recombinant p15E of FeLV-A, strain Glasgow 1, and the corresponding sequence of an endogenous p15E are given at the top. Cats 14, 34 and 44 were immunized with p15E while the others are representative of FeLV-infected cats. Strong epitopes are marked in black, weak epitopes in gray. Common groups of epitopes are framed (E1a, E1b, E2a, E2b). In addition, epitopes recognized by the serum from goat 27, immunized with p15E, and consensus epitopes recognized by 8 rats immunized with p15E are shown (hatched). For comparison, the C-terminal part of the HIV-1 transmembrane envelope protein gp41 and the localization of epitopes recognized by two monoclonal antibodies (mAb) broadly neutralizing HIV-1, (2F5 and 4E10) are shown (framed).

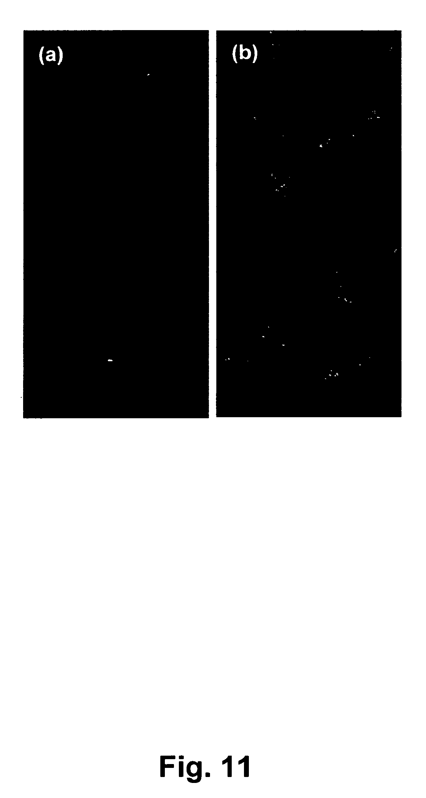

Fig. 11: Indirect immunofluorescence, visualized by confocal laser microscopy, using antiserum from cat 44 (immunized with p15E) and FITC-conjugated anti-cat IgG. FITC staining was measured at 488nm and unspecific fluorescence measured at 543nm was subtracted. (a) Uninfected FEA cells, (b) FeLV-A producing FEA cells.

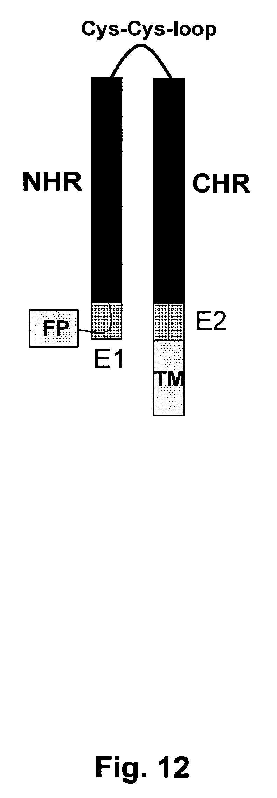

Fig. 12: Schematic representation of the transmembrane protein of retroviruses at a defined stage of infection. After introduction of the fusion peptide (FP) the N-terminal helical region (NHR) and the C-terminal helical region (CHR), which are connected by a cysteine-cysteine loop (Cys-Cys-loop), interact, bringing the epitope regions E1 and E2 into close proximity. TM indicates the transmembrane domain of the protein.

### Example 1

Affinity-purified recombinant fusion protein p15E of FeLV-A, strain Glasgow, was produced and characterized as described by Langhammer et al. (2005), Vaccine 23, 3341-3348.

Briefly, the ectodomain (aa 476-583) of p15E of FeLV-A was cloned into, the pCal-n vector (Stratagene, Europe, Amsterdam, Netherlands), expressed in *E. coli* BL21 DE3 cells, and the fusion protein containing p15E N-terminally fused to a 4kDa calmodulin binding protein (CBP) was purified by calmodulin resin affinity chromatography (Stratagene). Protein to be used for immunization was extensively dialyzed against phosphate-buffered saline (PBS).

Two immunization experiments were performed using Wistar rats. In the first experiment, 9 rats were immunized twice intramuscularly (i.m.) and subcutaneously (s.c.) (at weeks 0 and 3). In the first experiment Leucogen, containing 0.1 mg p45 plus Quil-A and aluminum hydroxide as adjuvant (Virbac, lot number 80986902143521) was given alone or mixed with p15E. Freund's adjuvant was used when p15E was injected alone. In the second experiment, 18 rats were immunized i.m. and s.c. at week 0 and 3 (Table 1). Immunization with p15E alone was performed at a dilution 7:3 in Montanide ISA 720 (Seppic, France, lot number 143521).

FeLV-A p15E and Leucogen p45-specific antibody titers were determined by ELISA. Briefly, ELISA plates (Nunc) were coated for 1 h at 37°C with affinity-purified recombinant p15E protein diluted in PBS or for 18h at 37°C with Leucogen p45 vaccine component diluted in PBS (100ng/well). Then, ELISA plates were washed one time with PBS containing 0.1 % Tween 20 and blocked for 1 h at room temperature with PBS containing 0.1% Tween 20 and 5% BSA. Serum samples diluted with PBS containing 2.5% BSA and 0.1 % Tween-20 were added to the ELISA plate at a starting dilution of 1:1000, diluted further (four-fold dilution series) and incubated for 1 h at 37°C. Then, ELISA plates were washed three times with PBS containing 0.1% Tween 20. A horseradish peroxidase conjugated secondary antibody specific for rat IgG (Bethyl, USA) diluted 1:3,500 with PBS containing 2.5% BSA and 0.1 % Tween-20 was used to detect antigen-specific immunoglobulin. Incubation for 1 h at 37°C was followed by washing five times with PBS containing 0.1% Tween 20. Finally, ELISA plates were developed by addition of OPD (alpha-phenylenediamine dihydrochloride) diluted in PBS (50µg/well) plus 0.1% H₂O₂ followed by inactivation with 30µl H₂SO₄ (5N) after 10 minutes. Protein-specific antibody endpoint titers are reported as the dilution giving an OD_{492/620nm} reading above that of preimmune sera.

When, in the first experiment, rats were immunized twice with 500µg p15E (group 50), 100µg Leucogen (group 51) or a mixture of Leucogen and p15E (group 52), all sera showed strong ELISA reactivity specific for the corresponding antigen used for immunization (Fig. 2a, b). Interestingly, the titer of binding antibodies specific for p45 was lower when Leucogen and p15E were injected simultaneously (group 52) in comparison to immunization with Leucogen alone (group 51) (Fig. 2b). When this experiment was repeated immunizing with 500µg p15E (group 55), 100µg p45 (group 56) and a mixture of both (group 54), ELISA titers of up to 4x10⁶ were observed against the corresponding antigen used for immunization (Fig. 3a, b, d, e; Fig. 4a, b). Again, mixing of p45 and p15E decreased the antibody response to p45 in two cases, animals 54.1 and 54.2 when compared with animals that received only p45 (animal group 56) (Fig. 4b). Firstly, the addition of p15E with its known immunosuppressive properties may have reduced the production of antibodies specific for p45. Alternatively, the addition of 500µg p15E to only 100µg p45 may have led to an antigenic dominance of p15E, or the addition of p15E to p45 may have led to interactions between domains of these two proteins, hiding epitopes of p45 from the immune system. *In vivo,* three transmembrane envelope proteins and three surface envelope proteins interact when building up the so-called knobs on the virus surface.

To investigate this further, Leucogen and p15E were injected at different sites (group 53) and the results were compared with the injection of a mixture at one site (group 54) (Fig. 4d, e) as had been performed in the first experiment (group 52) (Fig. 4b). Since in two cases (animals 54.1 and 54.2, Fig. 4e) the titer of the binding antibodies specific for p45 was reduced in comparison to all sera of group 53, it seems likely that the simultaneous injection of both antigens when injected into a single site was responsible for the decrease in antibody response.

To study the influence of the amount of p15E antigen on the induction of antibodies specific for p15E, injection of 500µg p15E (group 55) was compared with injection of 100µg p15E (group 57) (Fig. 5d). There was no obvious difference in the titers of binding antibodies as measured by ELISA. To investigate whether higher amounts of p15E in the antigen mixture has any influence on the antibody induction specific for p45, Leucogen vaccines containing 100µg p45 were injected together with 500µg p15E (group 54) or together with 100µg p15E (group 60, Fig. 5b). There was no obvious effect of the increased p15E amounts on the production of binding antibodies specific for p45 or p15E.

The virus stock for the neutralization assay was prepared as cell-free supernatant from feline embryonic fibroblast (FEA) cells infected with the FeLV-A Glasgow strain. The stock was titrated on uninfected FEA cells and was shown to have a titer of 10^{4.76} TCID₅₀/ml. Neutralization assays were performed as follows: One day before the assay, uninfected FEA cells were seeded at 6000 cells per well into 96-well microtiter plates. Preimmune and immune sera were heat-inactivated at 56°C for 30 min. 50µl of stock virus were incubated with four-fold serial dilutions of serum for 30 min at 37°C and than transferred to the cells. After 3 days incubation, cells were freeze-thawed three times and a lysis buffer containing 20 mg/ml of proteinase K in PCR buffer (50 mM KCI, 1.5 mM MgCl₂, 10 mM Tris-HCL, pH 8.4) was added. The cells were incubated for 3h at 56°C followed by 10 min at 95°C to inhibit proteinase K activity. Proviral DNA was measured by real time PCR as described below.

An internal probe FAM-5'-TTAAGCACCTGGGCCCCGGC-3'-DQ (Eurogentec) was used together with FeLV-specific primers. The sense primer 5'-TCAAGTATGTTCCCATGAGATACAA-3' and antisense primer 5'-GAAGGTCGAACTCTGGTCAACT-3' were used to amplify and to quantify a 185bp product from the exogenous U3 sequence in the LTR region of the FeLV-A provirus genome. The 25µl reaction mixture consisted of 1 x PCR buffer with 1mM MgCl₂, 0.5µM each of dATP, dCTP, dGTP, dTTP, 5pmol of each primer, 5pmol of probe, 1.25 U Amplitaq Gold polymerase and 2µl lysis mixture. The thermal cycling conditions used were 12 minutes at 95°C followed by 50 cycles of 1 minute at 95°C, 1 minute at 59°C and 30 seconds at 72°C in a Stratagene MX4000 machine.

When tested for neutralizing activity, 14 out of 18 antisera induced in the second experiment were able to inhibit the infection of FEA cells by FeLV-A with varying efficacy, whereas all preimmune sera had no such neutralizing activity (Fig. 3c, f; Fig. 4c, f; Fig. 5c, f). Sera from animals 55.1, 55.3, 57.2, and 57.3 did not show neutralizing activity. To analyze the neutralization efficacy, serial dilutions (1:4, 1:16 and 1:64) of the antisera were tested.

Antisera generated by immunizing with 500µg p15E (group 55) showed neutralization of FeLV-A ranging from nearly 0% (55.1 and 55.3) to 80% (55.2) at a 1:4 dilution (Fig. 3c). None of these sera had the ability to neutralize the virus at the final serum dilution of 1:64. This confirmed previous data showing the induction of neutralizing antibodies after immunizing rats with 500µg p15E. Whereas in the previous report Freund's adjuvant was used, Montanide was employed as adjuvant in the present investigation. However, due to the small number of animals the influence of the adjuvant cannot be analyzed. When the amount of p15E antigen used for immunization was reduced to 100µg (group 57), no reduction of the titer of neutralizing antibodies was observed (Fig. 3f). One antiserum (57.1) neutralized 90% of virus at a serum dilution of 1:4, but did not neutralize at 1:64. Two other antisera from animals of this group, 57.2 and 57.3, did not neutralize at any serum dilution.

Antisera obtained after immunization with 100µg Leucogen alone (group 56) neutralized at a range from 80% to 100% at a dilution of 1:4 and did not neutralize at 1:64. These data show that the neutralizing capacity *in-vitro* is much higher after immunization with Leucogen when compared with immunization with 500µg (Fig. 3c, group 56 versus 55) or 100µg p15E (Fig. 3f, group 56 versus 55).

When the neutralizing capacity of the sera obtained after simultaneous immunization with Leucogen and p15E (group 54) was compared with the neutralizing capacity of sera obtained with Leucogen alone (group 56), better neutralization was observed when both antigens were injected (Fig. 4c). Interestingly, the increase in neutralizing activity was associated with a decrease in the titer of binding antibodies specific for p45 (Fig. 4b).

However, when Leucogen and p15E were injected at different injection sites (group 53), the titers of binding antibodies specific for p45 was not reduced (Fig. 4e). This means that the titers of binding antibodies specific for p45 were comparable with titers in the sera from animals which received Leucogen alone (group 56, Fig. 4b) and the neutralizing capacity was as high as in the sera from animals which received simultaneously Leucogen and p15E at a single injection site (group 54, Fig. 4f). In the case that 500µg p15E and 100µg p45 were separately injected at different injection sites (group 53), neutralization efficacy ranging from 100% at a serum dilution of 1:4 to about 75% or more at a serum dilution of 1:64 were observed (Fig. 4f). The antisera obtained after immunization using a single injection site for 500µg p15E and 100µg p45 (group 54) showed neutralization efficacy ranging from 100% at a serum dilution of 1:4 to about 80% or more at a serum dilution of 1:64 (Fig. 4c, 4f). This indicates that the titer of neutralizing antibodies is not affected by the addition of p15E to p45 despite the finding that the titer of binding antibodies specific for p45 was reduced in two cases (Fig. 4e).

To evaluate the influence of the amount of p15E on the antibody response against p45, animals were immunized with Leucogen either together with 500µg (group 54) or together with only 100µg p15E (group 60) (Fig. 5). The binding antibody response specific for p15E was slightly lower in the group that received only 100µg p15E (Fig. 5a). As already mentioned above, the binding antibody response specific for p45 was identical in both groups (Fig. 5b). In contrast, the neutralizing capacity was significantly higher in sera from animals that received 500µg p15E (Fig. 5c), indicating that higher amounts of p15E induced more neutralizing antibodies in connection with Leucogen. When 500µg (group 55) or 100µg p15E (group 57) were applied without Leucogen, no differences in the binding antibody response specific for p15E (Fig. 5d) and in the neutralizing capacity (Fig. 5f) were observed.

An epitope mapping of the induced sera was performed using a cellulose-adsorbed peptide spot library of linear 15-mer peptides overlapping by 13 amino acids. The peptides corresponding to the entire p15E of FeLV-A Glasgow strain were covalently bound to the cellulose membrane by the C-terminus (Fig. 6). A standard protocol was applied for synthesis (Jerini Biotools). Sera diluted 1:1000 were incubated with the membrane for 3h, washed three times for 15 min with Tris-buffered saline, pH 7.5 containing 0.05% Tween 20 (Sigma) and incubated for 2 h with a peroxidase-conjugated secondary antibody diluted 1:10,000. Binding was detected using a chemiluminescence detection solution (ECL, Amersham Pharmacia Biotech).

When sera from rats immunized with recombinant p15E alone (groups 55, 57) were analyzed, four main epitope regions were found, two at the N-terminus and two at the C-terminus of the ectodomain (Fig. 7). These findings confirmed previous studies, in which the same epitope regions were identified and were designated E1a (LETAQFRQL) and E1b (IQALEESISALEK) as well as E2a (KQRQQLF) and E2b (FDSQQGWFEGWFN). In contrast to the previous study, the E2a epitope was better defined (KQRQQLF instead of MAKLRERLKQRQQL). These four epitopes were identified, regardless of whether 500µg (group 55) or 100µg (group 57) of p15E were applied (Fig. 7). Interestingly, none of the non-neutralizing sera recognized the E1 epitopes, suggesting that this epitope is essential for neutralization.

The same epitopes were identified when rats were immunized with p15E and Leucogen (animal groups 53, 54 and 60), indicating that Leucogen did not change the recognition of the epitopes by the immune system (Fig. 7). It was also shown that the adjuvant does not influence the recognition of the epitopes: regardless of whether Freund's adjuvant, Montanide (groups 55 and 57) or the adjuvant contained in the Leucogen preparation (groups 53, 54 and 60) were used, the same epitopes were recognized.

As expected, no p15E specific epitopes were detected when sera were tested from rats immunized with Leucogen p45 alone (group 56), indicating that the Leucogen vaccine does not contain parts of the transmembrane envelope protein.

Sera induced with 100µg or 500µg p15E (groups 55 and 57) always detected the E2b epitope, while the E1 b epitope was recognized only by one antiserum of each group (55.2 and 57.1). However, only these two antisera efficiently neutralized FeLV (Fig. 5f), indicating a critical role of the E1 b epitope in virus neutralization. The E2 epitopes also seem to be crucial for neutralization since the serum from animal 60.1 did not recognize E2 and had only a weak neutralization activity when compared with the sera from two other animals of the same group that recognize E2 (Fig. 5c). As mentioned above, only two of the sera from animals immunized with p15E recognized E1 b and only these sera were neutralizing. On the other hand, sera from all animals immunized with p15E and Leucogen recognized E1 b (Fig. 7), indicating that simultaneous immunization of both antigens increased the recognition of the crucial epitope E1 b.

### Example 2

For immunization recombinant p15E of FeLV-A was prepared as described above.

6-10 month old cats, obtained from the University of Düsseldorf and housed in groups of 3, were immunized intramuscularly (i.m.) twice (at weeks 0 and 3) with p15E (Table 2). Montanide ISA 720 (Seppic, France, lot number 143521) was used as adjuvant mixed with p15E at a ratio of 3:7.

SDS-PAGE and Western blotting were performed as described by Tacke et al. (2001), Xenotransplantation 8, 125-135, using 1µg of the affinity purified recombinant FeLV p15E per lane. In Western blot analyses, antisera from all three cats immunized with 500µg p15E (#14, #34, and #44) specifically detected the recombinant p15E protein at a size of 15kDa, while the preimmune sera did not react (Fig. 8a). When sera from FeLV-infected housecats were tested in the same assay, 44 of 75 sera (58.6%) also specifically detected p15E, four of which are shown in Fig. 8a. Sera were obtained from household cats in Germany at the time of first diagnosis of infection using a commercial p27 Gag antigen detection assay (Feline leukemia virus antigen test kit, Symbiotics, USA). These data indicate that immunized and infected animals are able to produce antibodies specific for p15E.

Pre- and post-immunization cat sera were titrated in ELISA as performed above. The same affinity-purified recombinant p15E used for immunization as in example 1 was applied as antigen herein. All three immune sera, but not the preimmune sera, strongly reacted in ELISA using recombinant p15E as antigen (Fig. 8b). After the boost immunization the titer of binding antibodies increased markedly (Table 2). Sera from cat 44 showed the highest titers of binding antibodies in this group (2.56 x10⁵ after the first immunization and 1x10⁶ after the boost). The sera from cats 14 and 34 showed titers of 6.4x10⁴ that increased to 2.56 x10⁵ after the boost immunization. In contrast, sera obtained from FeLV-infected housecats had only titers between 1x10³ and 4x10³ (Table 2).

The virus neutralization assay and real time PCR were performed as already described with the following exception: 75µl of stock virus were incubated with four-fold serial dilutions of serum (previously heat-inactivated at 56°C for 45 min) for 45 min at 37°C and than transferred to the cells. Neutralization of FeLV-A strain Glasgow infection of feline embryonic fibroblast cells was measured using four-fold serial dilutions (from 1:16 to 1:16384) of the sera. All sera taken after the first immunization had titers of 1:256 (Fig. 9) whereas no preimmune sera showed neutralizing activity. Similar to the titers of binding antibodies, the titers of neutralizing antibodies increased after the booster immunization in two animals (cats 14 and 34) up to 1:1024 (Table 2) although the titer of neutralizing antibodies in the serum of cat 44 did not increase.

To identify the epitopes recognized by the immune sera, epitope mapping was performed according to example 1 (Fig 10a). Four major epitopes were identified (Fig. 10b) using sera from the cats immunized with p15E (#14, #34, and #44). The first epitope, KALLETAQF, is nearly identical to an epitope identified by immunizing a goat with FeLV p15E and to a consensus epitope (LETAQFRQL) recognized by sera from 8 rats immunized with the same antigen. This epitope group was designated E1a. The second epitope (ALEESISALEK, E1 b) was also recognized by all 8 rat sera but not by the goat serum. The third epitope is located in the immunosuppressive domain, LQNRRGLDILFLQEGGL, which is highly conserved amongst all retroviruses. Synthetic peptides corresponding to this domain inhibit lymphocyte proliferation and modulate cytokine production. This epitope in the immunosuppressive domain was also recognized by the goat serum, but not by any of the rat sera. The fourth epitope, MAKLRERLKQRQQLF, corresponds to an epitope E2a, recognized both by the goat serum and by 7 of 8 rat sera. Similar to the goat serum the cat sera did not recognize an epitope recognized by all rat sera and designated E2b (FDSQQGWFEGWFN). The cat sera bind to main epitopes described following immunization of rats and goats. These data support the existence of main target epitopes after immunization with p15E and minor species-specific differences.

Sequences homologous to the epitopes are also present in endogenous retroviruses. When the sequence of the FeLV-A p15E used for immunization was compared with that of the endogenous feline retrovirus CFE-6 (NCBI accession no. gi:74706), sequence homologies were identified in the epitope domains (Fig. 10b). Despite such sequences being present in the genomes of the immunized cats, binding and neutralizing antibodies specific for these domains were induced.

To elucidate the possible mechanisms of neutralization, the localization on the cell surface of the epitopes recognized by the p15E-specific sera were analyzed by immunofluorescence using non-permeabilized FeLV-infected FEA feline embryonic fibroblast cells. FeLV-A producing FEA cells were grown on chamber slides, washed three times with PBS and fixed with 3.5% formaldehyde. Unspecific binding sites were blocked with 5% BSA in PBS for 20 minutes followed by washing with PBS. Cat sera were applied in 2.5% BSA/PBS at a dilution of 1:1000 and incubated at 37°C for 1 h. After five washes with PBS the cells were incubated with FITC-labeled goat anti-cat IgG (Bethyl, USA). Finally, the cells were embedded in Prolong antifade reagent (Molecular Probes) and the surface fluorescence was analyzed by confocal microscopy (Zeiss, LSM510). Unspecific cell fluorescence at 543nm was subtracted from the specific signal at 488nm. Uninfected cells were not recognized by cat sera #14, #34, and #44 were used (Fig. 11 a). However, all three bound to the cell surface (Fig. 11 a) whereas the corresponding preimmune sera did not. To increase picture quality, unspecific cell fluorescence at 543nm was subtracted from FITC-specific signal at 488nm. This binding of immune sera to the cell surface indicates that the epitopes identified are accessible to FeLV on the surface of infected cells.

To compare neutralizing antibody responses in FeLV-infected cats with those of p15E-immunised animals, sera from infected housecats were analyzed. As described above, 44 of 75 sera investigated showed antibodies specific for p15E by Western blot analysis (Fig. 8a), and the titers of p15E-specific antibodies in ELISA ranged from ≤1 x10³ to 4 x10³ (Table 2). Neutralizing titers of these sera were found to be between 0 and 1:256 (Table 2), although it must be kept in mind that in the infected cat neutralizing antibodies might also be directed against other viral proteins such as gp70.

Epitope mapping using overlapping peptides spanning the entire p15E (Fig. 10) was carried out. Serum from cat 9425 only recognized the epitope E2a, while serum from cat 6452 recognized an epitope located outside E1a as well as E1b and E2a, and serum from cat 27047 recognized the epitopes E1a and E1b weakly, but E2a more strongly. Cat 27047 had initially been immunized with Leucogen containing the non-glycosylated surface envelope protein p45, but became infected despite this immunization. Sera from cats 54748 and 55409 (Fig. 10a) weakly detected epitopes E2a and E2b, but none of these epitopes were recognized by serum from cat 55284, despite this cat being infected and having low titer neutralizing antibodies.

**Table 1 Titers of binding and neutralizing antibodies after immunization with Leucogen, p15E or both antigens**

| Group | No. of animals | Antigen(µg) | | Application site for combined immunizations | Adjuvant^{a)} | Titers of antibodies specific for p15E(ELISA) | Titers of antibodies specific for p45(ELISA) | Neutralization Titer | Number of sera with neutralizing activity at dilution 1:4 | Number of sera with neutralizing activity at dilution 1:64 |
|---|---|---|---|---|---|---|---|---|---|---|
| | | p15E | Leucogen | | | | | | | |
| | 53.1 | | | | LA and | 1x10⁶ | 6.4x10⁴ | 1:64 | | |
| 53 | 53.2 | 500 | 100 | Two separate | Montanide | 1x10⁶ | 6.4x10⁴ | 1:64 | 3 | 3 |
| | 53.3 | | | | | 1x10⁶ | 6.4x10⁴ | 1:64 | | |
| | 54.1 | | | | | 1x10⁶ | 1.6x10⁴ | 1:64 | | |
| 54 | 54.2 | 500 | 100 | Single | LA | 2.56x10⁵ | 1.6x10⁴ | 1:64 | 3 | 3 |
| | 54.3 | | | | | 1x10⁶ | 6.4x10⁴ | 1:64 | | |
| | 55.1 | | | | Montanide | 1x10⁶ | - | 0 | | |
| 55 | 55.2 | 500 | - | - | | 4x10⁶ | - | 1:16 | 1 | 0 |
| | 55.3 | | | | | 4x10⁶ | - | 0 | | |
| | 56.1 | | | | | - | 6.4x10⁴ | 1:16 | | |
| 56 | 56.2 | - | 100 | - | LA | - | 6.4x10⁴ | 1:16 | 3 | 0 |
| | 56.3 | | | | | - | 6.4x10⁴ | 1:16 | | |
| | 57.1 | | | | Montanide | 4x10⁶ | - | 1:16 | | |
| 57 | 57.2 | 100 | - | - | | 1x10⁶ | - | 0 | 1 | 0 |
| | 57.3 | | | | | 4x10⁶ | - | 0 | | |
| | 60.1 | | | | | 2.56x10⁵ | 1.6x10⁴ | 1:16 | | |
| 60 | 60.2 | 100 | 100 | Single | LA | 2.56x10⁵ | 1.6x10⁴ | 1:64 | 3 | 2 |
| | 60.3 | | | | | 2.56x10⁵ | 6.4x10⁴ | 1:64 | | |

| | | | | | | | | | | |
|---|---|---|---|---|---|---|---|---|---|---|
| ^{a)}LA - Leucogen adjuvant containing Quil-A and aluminum hydroxide, Montanide- Montanide ISA 720 | | | | | | | | | | |

**Table 2 Characterization of sera from immunized and FeLV- infected cats**

| Serum | Immunization | FeLV diagnosis | Western blotting p15E | ELISA titre¹ | Neutralization titer² | Epitope mapping³ | | | |
|---|---|---|---|---|---|---|---|---|---|
| | | | | | | E1a | E1b | E2a | E2b |
| 14 | p15E | - | + | (6.4x10⁴) 2.56x10⁵ | (1:256) 1:1024 | ++ | + | ++ | - |
| 34 | p15E | - | + | (6.4x10⁴) 2.56 x10⁵ | (1:256) 1:1024 | ++ | + | ++ | - |
| 44 | p15E | - | + | (2.56 x10⁵) 1 x10⁶ | (1:256) 1:256 | ++ | + | ++ | - |
| 9425 | none | + | - | ≤1 x10³ | 1:16 | - | - | -/+ | - |
| 6452 | none | + | + | 4 x10³ | 1:256 | - | ++ | ++ | - |
| 27470 | various vaccines | + | -/+ | 4 x10³ | 1:256 | + | + | -/+ | - |
| 54748 | none | + | + | 4x10³ | 0 | - | - | + | + |
| 55409 | none | + | -/+ | 4x10³ | 1:64 | - | - | + | + |
| 55284 | none | + | - | ≤1x10³ | 1:64 | - | - | - | - |

| | | | | | | | | | |
|---|---|---|---|---|---|---|---|---|---|
| 1: ELISA using recombinant p15E, titers after the booster immunization (titers obtained after first immunization are shown in brackets) 2: Inhibition of provirus integration measured by real time PCR in comparison to preimmune sera, titers after the booster immunization (titers obtained after first immunization are shown in brackets) 3: ++ strong detection, + weak detection, - no detection | | | | | | | | | |

## Claims

1. Pharmaceutical combined composition comprising as active ingredients an effective amount of the retroviral transmembrane envelope protein p15E, gp41 or gp36, and an effective amount of a retroviral surface envelope protein, or parts thereof, or the DNA encoding said proteins, whereby said proteins are contained as single molecules.

2. Composition according to claim 1, wherein the transmembrane envelope protein and the surface envelope protein are of the same retroviral origin.

3. Composition according to claim 1 or 2, wherein said proteins are originated from FeLV, PERV, KoRV, HIV-1 or HIV-2, preferably FeLV.

4. Composition according to claim 1, wherein p15E comprises an epitope E2 between membrane passage and C-terminal helical region, whereby the E2 epitope is selected from a region between membrane passage and C-terminal helical region of a retroviral transmembrane envelope protein which is not identical to p15E.

5. Composition according to claim 4, wherein p15E comprises E2 of HIV-1 gp41.

6. Composition according to one of claims 1 to 5, wherein the surface envelope protein is gp70, gp120 or an unglycosylated form thereof.

7. Composition according to claim 6, wherein the surface envelope protein is p45.

8. Composition according to one of claims 1 to 7, wherein the part of the transmembrane envelope protein is the ectodomain.

9. Composition according to claim 8, wherein the ectodomain comprises the amino acid sequences E1a (LETAQFRQL), E1b (IQALEESISALEK), E2a (KQRQQLF) and/or E2b (WFEGWFN).

10. Composition according to one of claims 1 to 9 comprising pharmaceutically acceptable excipients.

11. Use of an effective amount of the retroviral transmembrane envelope protein p15E, gp41 or gp36, and an effective amount of a retroviral surface envelope protein, or parts thereof, or the DNA encoding said proteins, whereby said proteins are contained as single molecules, for the manufacture of a pharmaceutical combined composition for the prophylactic or therapeutic treatment and/or monitoring of retroviral diseases, preferably retroviral immune diseases.

12. Use according to claim 11 for the prophylactic or therapeutic treatment and/or monitoring of a FeLV infection.

13. Use according to claim 11 to 12 as an injection solution.

14. Use according to one of claims 11 to 13, wherein the two active ingredients are administered simultaneously or sequentially.

15. Neutralizing antibodies produced by immunization with the composition according to one of claims 1 to 10.

16. Method for treating a retroviral disease, preferably a FeLV infection, wherein as active substances an effective amount of the retroviral transmembrane envelope protein p15E, gp41 or gp36, and an effective amount of a retroviral surface envelope protein, or parts thereof, or the DNA encoding said proteins, are administered to a mammal in need of such treatment, whereby said proteins are administered as single molecules.

17. Method for inducing an antibody response, wherein the composition according to one of claims 1 to 10 is administered to a mammal, thereby inducing the production of neutralizing antibodies.

18. Method according to claim 17, wherein the composition is injected.

19. Method for passive immunization of a mammal, wherein the antibodies induced by the method according to claim 17 are separated and administered to a mammal.
